# EUROPEAN PATENT SPECIFICATION

(11) **EP 2 085 091 B1**
(45) Date of publication and mention of the grant of the patent: **07.09.2011**
(21) Application number: 07800884.4
(22) Date of filing: 07.09.2007
(51) Int. Cl.: A61K 33/20, A61P 3/10, A61P 13/08

(54) **USE OF HYDROCHLORIC ACID IN MANUFACTURE OF MEDICAMENT FOR TREATING DIABETES**
VERWENDUNG VON SALZSÄURE BEI DER HERSTELLUNG EINES MEDIKAMENTS ZUR BEHANDLUNG VON DIABETES
UTILISATION D'ACIDE CHLORHYDRIQUE DANS LA FABRICATION DE MÉDICAMENT DESTINÉ AU TRAITEMENT DE DIABÈTE

(30) Priority: 08.09.2006 CN 200610053309
(43) Date of publication of application: 05.08.2009
(73) Proprietor: Liu, Zhiren, Hangzhou, Zhejiang 310006 (CN)
(72) Inventor: Liu, Zhiren, Hangzhou, Zhejiang 310006 (CN)
(74) Representative: ABG Patentes, S.L.
(86) International application number: PCT/CN2007/002668
(87) International publication number: WO 2008/043243

(56) References cited:
- WO-A1-2004/039148
- WO-A2-02/080940
- WO-A2-03/028623
- DATABASE WPI Week 200282 Thomson Scientific, London, GB; AN 2002-751463 XP002559862 & CN 1 357 333 A (FENG W) 10 July 2002 (2002-07-10)
- DATABASE WPI Week 200472 Thomson Scientific, London, GB; AN 2004-732061 XP002559863 & JP 2004 285034 A (NAGAI M) 14 October 2004 (2004-10-14)

## Description

### FIELD OF THE INVENTION

The present invention relates to the use of hydrochloric acid and particularly to the use of dilute hydrochloric acid to manufacture a medicament for treating diabetes

### BACKGROUND OF THE INVENTION

The cause and pathogenesis of diabetes are very complicated, and are not fully understood so far, which according to literature disclosed by IDF (International Diabetes Federation) and WHO (World Health Organization) in 1999. But, at present, traditional theory and modern medicine both believe that blood glucose level is closely related to the secretion of insulin and the amount and species of diets. Medical professions also believe that diabetes has no radical cure by far, and can only be controlled by following measures, i.e. diet control, oral administration of hypoglycemic agents, injection administration of insulin, or oral administration of hypoglycemic agents in combination with injection administration of insulin..

### DISCLOSURE OF THE INVENTION

### Problem to be solved by the present invention

At present, blood glucose control is the main target for treating the above mentioned diabetes, either lowering the intake of glucose, or artificially increasing of insulin level is adopted to make glucose smoothly decomposed. But no matter what method is adopted, lifelong drug administration or insulin injection and diet control are indispensable. In addition, those treatment methods can only be effective as to control the deterioration rate of diabetes and its complications, however, the disease will be getting worse along with aging.

For solving the aforementioned issues, based on research and practice in many years, the inventor of the present invention has found that the formation of diabetes

is based on the following mechanism, i.e. adhesives calcium carbonate and calcium oxalate, which are generated from calcium bicarbonate in water and food via decomposition, adhere human body metabolites (such as cholesterol crystal, etc.) and solid matter in human body to human organ and the inner wall of cardiac blood vessel used for transporting various components and metabolites, which prevents transporting, even causes blockage of transportation path, further leads to inflammation, and finally results in the formation of diabetes.

From the above mechanism, it can be learned that the cause of diabetes is that:
1. Adhesives calcium carbonate and calcium oxalate, which are generated from calcium bicarbonate in water and food via decomposition, adhere human body metabolites (such as cholesterol crystal, etc.) and solid matter in human body to the inner wall of capillary blood vessel used for transporting blood glucose and to the cell entrance used for blood glucose to enter into cell, and thus form fouling (calcification) in capillary blood vessel and cell entrance. With aging, the fouling on the inner wall of capillary blood vessel and the cell entrance gets thicker, and the fouling area gets larger, both of which prevent a large amount of blood glucose and other nutritional substance from entering into and being consumed by cells; therefore, a large amount of blood glucose and other nutritional substance retain in blood to cause diabetes. This type of diabetes has been known as Type II diabetes.
2. Adhesives calcium carbonate and calcium oxalate, which are generated from calcium bicarbonate in water and food via decomposition, adhere human body metabolites (such as cholesterol crystal, etc.) and solid matter in human body to pancreas and to the tract wall used for transporting insulin. When the fouling accumulates to certain extent, following three conditions may occur:
   (1) A large amount of fouling accumulates in pancreas, however the tract used for transporting insulin is unblocked; due to the fouling in pancreas, this negatively affects insulin secretion amount, and the secreted insulin activity is poor.
   (2) Pancreas is intact, however, the insulin transporting tract accumulates fouling, and thus insulin secreted by pancreas is contaminated when passing through the blocked transportation tract, and therefore, weakened the capability for transporting blood glucose.
   (3) Pancreas and insulin transportation tract both accumulate fouling, the amount of insulin transported to blood is less, and the insulin activity is poor.

Insulin is a substance for carrying blood glucose while entering into cell for being consumed; due to the deficiency of carrying vehicle and its poor activity, any one of the above problems will prevent a large amount of blood glucose which should be absorbed and consumed by cells, from entering into cells for being absorbed and consumed, and thus leads to blood glucose elevation and over nutrition in the blood. When blood glucose is excess the normal value, this will results in diabetes. This type of diabetes has been known as Type I diabetes.

Based on the above analysis, the inventor of the present invention deems that the present treatment measures can only alleviate symptoms or delay deterioration rate of diabetes, but can not radically cure the disease.

From explanation of above symptoms, the inventor has found and deemed that all the organs and tracts with transportation function in human body will generate fouling, once fouling accumulates, a series of diseases will arise, for example, acquired obstruction of spermaduct and oviduct, also calculus, onychomycosis, gout, senile plaque, breast lobule hyperplasia, dull skin, gynaecopathia, hemorrhoids and hyperostosis, etc. The functions can get back to normal as long as fouling on the transportation duct wall, inner wall of secretory gland, cell entrance, and capillary blood vessel wall is removed. To remove the fouling, adhesives calcium carbonate and calcium oxalate must be dissolved, and then other attached small particulate matters can be separated and excreted out of the body.

In other words, the present invention aims to solve the above problems, and to provide a substance applied in medicament for treating human organ diseases caused by fouling of human organs and of transportation tract in human body.

Specifically, the object of the present invention is to provide a medicament for treating diabetes. More specifically, the invention relates to the use of hydrochloric acid to manufacture a medicament for the treatment of diabetes.

### Method for solving the problems

Based on the aforementioned mechanism, after research in depth, the inventor has found that hydrochloric acid is the most ideal substance for dissolving calcium carbonate and calcium oxalate in human body, at the same time, hydrochloric acid can be adsorbed by the protection membrane on inner wall of the cells, and repair damaged blood vessels and pancreas.

Therefore, for solving the above issues, the present invention provides a medicament using hydrochloric acid for treating human organ diseases caused by fouling of human organs and of transport tract in human body.

The present invention further provides a medicament comprising hydrochloric acid for treating

Additionally, the volume concentration of hydrochloric acid in the present invention is preferably 0.005%-0.1%.

The pH of the aqueous solution of hydrochloric acid in the present invention is preferably controlled within 1-6.9.

### Effect of the Present Invention

For explaining therapeutic effect of hydrochloric acid to diabetes in the present invention, 106 diabetes patients who are oral administration hydrochloric acid aqueous solution, are observed clinically, in which the number of male patients is 56, and the number of female patients is 50, in addition, the average age is 59, and the age range is 46-72. The practice result shows that the number of the diabetes patients using edible hydrochloric acid aqueous solution in combination with hypoglycemic agents, and the diabetes patients only using edible hydrochloric acid aqueous solution who are completely cured, is 45; the number of the diabetes patients using edible hydrochloric acid aqueous solution in combination with hypoglycemic agents, whose blood glucose index has been controlled within normal range and have reduced administration of hypoglycemic agents, is 31; the number of diabetes patients, whose blood glucose index unable to be controlled within normal range by oral administration of hypoglycemic agents and by injection insulin, after oral administration of edible hydrochloric acid aqueous solution for two years, have blood glucose index controlled within normal range by using same oral hypoglycemic agents and insulin injection, is 30.

The medicament prepared from hydrochloric acid in the present invention is safe and reliable for treating diabetes and has exact and significant effect, moreover capable of converting calcium salt harmful for human body into calcium benefit for human body.

### Embodiment

The present invention will be further explained through following embodiments. It should be understood that those embodiments are only used for explaining the present invention while not for limiting scope of the present invention, and other implementation manners included in the claim scope are also applicable.

In the following embodiments, traditional conditions or conditions recommended by manufacturers are adopted in the experiment methods unless specified otherwise.

### Embodiment 1

2% of high purity hydrochloric acid is added into drinking water, stirred well, regulated to pH of 1-6.9, and diluted to volume concentration (V/V) of 0.005%-0.1%; to be specific, the pH value can be regulated to 1, 6.9, 3, 6.5, 6.7, 4.5, or 2.0, and the volume concentration after dilution can be 0.005%, 0.1%, 0.015% or 0.01%. The prepared hydrochloric acid-containing water solution can be directly taken as edible hydrochloric acid solution. Subjects to be treated are administered with the hydrochloric acid solution at 100-3,000ml per day on average, preferably not less than 1,500ml by taking separately 3-6 times per day. 3-6 months is one treatment course, which will last about 0.5-6 years.

When tap water is adopted for preparing hydrochloric acid solution, purification device should be added at front end to make the tap water satisfy national drinking water standard.

The temperature of the edible hydrochloric acid solution in the present invention is preferably room temperature; basiclly heating is not allowed, for heating will volatilize hydrochloric acid and lost its efficacy. Beverage and tea incline to fouling are not recommended, for theine will neutralize hydrochloric acid and lost its efficacy.

### Embodiment 2

0.005% (V/V) hydrochloric acid aqueous solution with pH 6.9 is prepared into injection. When injected for treatment of diabetes, the recommended dosage is 500ml per day injected by two times in intravenous drip manner, and 2-3 months are one treatment course. By treating diabetes according to the above method and dosage, the dosage for oral administration of hypoglycemic agents and injection of insulin can be reduced by 1/4 after one year, reduced by half after two years, and reduced to maintenance dose or dose free after three years, and thus the blood glucose can be back to normal.

### Embodiment 3

High purity hydrochloric acid is added into drinking water to be diluted into 2% hydrochloric acid solution, and filled in container with flow rate control. The hydrochloric acid solution and drinking water are mixed at certain flow rate ratio via pipes, and pH detector is provided at the end of the pipes. pH of the hydrochloric acid solution is controlled within 4-6.9 to give 0.5% (V/V) edible hydrochloric acid solution.

15 diabetes patients administered with the above edible hydrochloric acid solution are clinically observed. The solution is expected administered at 2,000ml per day, in which 500ml is respectively administered one hour before breakfast and lunch, and 1,000ml is administered one hour before supper.

Table 1 shows the improvement of diabetes condition of partial patients after oral administration of the hydrochloric acid solution in the present invention.

| Table 1 treating diabetes with edible hydrochloric acid solution | | | |
|---|---|---|---|
| Patient No. | Fasting blood glucose (mmmol/L) | Age | Treatment effect of diabetes (in which the number is fasting blood glucose value) |
| 1 | 7.1 | 50 | respectively reduced to 6.7 and 6.1 after administration of hydrochloric acid solution for half year and one year |
| 2 | 7.3 | 52 | respectively reduced to 6.6 and 5.9 after administration of hydrochloric acid solution for one year and two years |
| 3 | 7.2 | 61 | respectively reduced to 6.8 and 6.2 after administration of hydrochloric acid solution for one year and two years |
| 4 | 7.1 | 69 | respectively reduced to 6.8 and 6.5 after administration of hydrochloric acid solution for one year and two years |
| 5 | 7.2 | 71 | respectively reduced to 7 and 6.6 after administration of hydrochloric acid solution for one year and two years |
| 6 | 6.1 (oral administration of hypoglycemic agent) | 46 | after administration of hydrochloric acid solution for half year, hypolycemic agent dosage is reduced by 1/2; after administration of hydrochloric acid solution for two years, administration of hypoglycemic agent is stopped, and fasting blood glucose value is reduced to 6.0. |
| 7 | 6.5 (oral administration of hypoglycemic agent) | 48 | after administration of hydrochloric acid solution for one year, hypolycemic agent dosage is reduced by 1/2; after administration of hydrochloric acid solution for two years, administration of hypoglycemic agent is stopped, and fasting blood glucose value is reduced to 6.1. |
| 8 | 6.7 (oral administration of hypoglycemic agent) | 53 | after administration of hydrochloric acid solution for one year, hypolycemic agent dosage is reduced by 1/3; after administration of hydrochloric acid solution for two years, administration of hypoglycemic agent is reduced by half; after administration of hydrochloric acid solution for three years, administration of hypoglycemic agent is stopped, and fasting blood glucose value is reduced to 6.2. |
| 9 | 6.8 (injection administration of insulin) | 66 | after administration of hydrochloric acid solution for one year, insulin injection dosage is reduced by 1/2; after administration of hydrochloric acid solution for two years, insulin injection is stopped, and fasting blood glucose value is reduced to 6.3 |
| 10 | 6.0 (oral administration of hypoglycemic agent, and injection administration insulin) | 59 | after administration of hydrochloric acid solution for one year, oral administration of hypoglycemic agent is stopped; after of 59 administration of hydrochloric acid solution for two years, insulin injection dosage is reduced by half, and fasting blood glucose value is reduced to 5.9. |
| 11 | 7.9 (oral admmistration of hypoglycemic agent) | 52 | Fasting blood glucose value is reduced to 6.9 after administration of hydrochloric acid solution for one year, the hypoglycemic agent dosage is reduced by half after administration of hydrochloric acid solution for 2 years, oral administration of hypoglycemic agent is stopped after administration of hydrochloric acid for three years, and fasting blood glucose value is reduced to 6.3. |
| 12 | 7.8 (oral administration of hypoglycemic agent) | 72 | Fasting blood glucose values are respectively reduced to 7.1 and 6.6 after administration of hydrochloric acid solution for one year and two years, the hypoglycemic agent dosage is reduced by half after administration of hydrochloric acid solution for 3 years, and fasting blood glucose value is reduced to 6.2. |
| 13 | 11 (oral administration of hypoglycemic agent) | 51 | Fasting blood glucose values are respectively reduced to 7.6 and 6.5 after administration of hydrochloric acid solution for one year and two years, the hypoglycemic agent dosage is reduced by half after administration of hydrochloric acid solution for 3 years, and fasting blood glucose value is reduced to 6.1. |
| 14 | 9.8 (oral administration of hypoglycemic agent, and injection administration of insulin) | 66 | after administration of hydrochloric acid solution for 1 year, oral administration of hypoglycemic agent is stopped, insulin injection dosage is kept the same, and fasting blood glucose value is reduced to 7.6; after administration of hydrochloric acid solution for 2 years, insulin injection dosage is reduced by 1/3, and fasting blood glucose value is reduced to 7. |
| 15 | 13 (oral administration of hypoglycemic agent, and injection administration of insulin) | 69 | Fasting blood glucose values are respectively reduced to 9.5 and 7.8 after administration of hydrochloric acid solution for one year and two years; after administration of hydrochloric acid solution for 3 years, the hypoglycemic agent dosage is reduced by half, and fasting blood glucose value is reduced to 6.5. |

Generally, administration dosages of oral hypoglycemic agent and/or insulin injection for diabetes patients are increased along with time. While it can be observed that after administration of edible hydrochloric acid solution in the present invention, the administration dosages of oral hypoglycemic agent and/or insulin injection can be decreased along with time, and the fasting blood glucose value can be effective controlled and reduced at the same time. The hydrochloric acid solution has significant effective for treating diabetes, with total effective rate of 100%.

### Embodiment 4 compound preparation of hydrochloric acid solution + diabetes medicinal preparation

One tablet of commercially available Tolbutamide (2.5mg) is added into 500ml hydrochloric acid solution with pH 6.9, to constitute compound preparation containing hydrochloric acid. Treatment effects similar to No. 6-8 in Table 1 can be obtained when the compound preparation is applied for treating diabetes.

Similarly, other compound preparations of hydrochloric acid + other diabetes medicament can be prepared.

It should be understood that those skilled in arts can modify or revise the present invention in various manners after the contents of the present invention are read. For example, the aqueous solution can be prepared from common drink water, such as physiologic saline, glucose solution, or juice etc; the preparation method is the same as aforementioned method, as long as pH of the oral liquid or injection is controlled. In other words, any aqueous solution, which won't react with hydrochloric acid to create chemical substances unbeneficial to human body, is applicable, and their equivalent falls into the scope of the present invention as well.

## Claims

1. Use of hydrochloric acid in the preparation of a medicament for the treatment of diabetes.

2. Use according to claim 1 **characterized in that** the volume concentration (V/V) of the aqueous solution of hydrochloric acid is 0.005%-0.1%.

3. Use according to claim 2 **characterized in that** the pH of the aqueous solution of hydrochloric acid is controlled within 1-6.9.

## Patentansprüche

1. Verwendung von Salzsäure bei der Herstellung eines Medikaments zur Behandlung von Diabetes.

2. Verwendung gemäss Anspruch 1, **dadurch gekennzeichnet, dass** die Volumenkonzentration (V/V) der wässrigen Lösung von Salzsäure 0,005 bis 0,1 % beträgt.

3. Verwendung gemäss Anspruch 2, **dadurch gekennzeichnet, dass** der pH-Wert der wässrigen Lösung von Salzsäure innerhalb von 1 bis 6,9 eingestellt wird.

## Revendications

1. Utilisation de l'acide hydrochlorique dans la préparation d'un médicament pour le traitement des diabètes.

2. Utilisation selon la revendication 1, **caractérisée en ce que** la concentration en volume (V/V) de la solution aqueuse d'acide hydrochlorique est de 0,005%-0,1%.

3. Utilisation selon la revendication 2, **caractérisée en ce que** le pH de la solution aqueuse d'acide hydrochlorique est maintenu entre 1 et 6,9.
